# EUROPEAN PATENT SPECIFICATION

(11) **EP 3 936 130 B1**
(45) Date of publication and mention of the grant of the patent: **14.02.2024**
(21) Application number: 21178882.3
(22) Date of filing: 06.05.2015
(51) Int. Cl.: A61K 31/473, A61P 25/00, A61P 25/14, A61P 43/00

(54) **VALBENAZINE DOSAGE REGIMEN FOR THE TREATMENT OF HYPERKINETIC MOVEMENT DISORDERS**
DOSIERUNGSSCHEMA FÜR VALBENAZIN ZUR BEHANDLUNG VON HYPERKINETISCHEN BEWEGUNGSSTÖRUNGEN
RÉGIME POSOLOGIQUE DE VALBÉNAZINE POUR LE TRAITEMENT DES TROUBLES DE MOUVEMENT HYPERKINÉTIQUE

(30) Priority: 06.05.2014 US 201461989240 P
(43) Date of publication of application: 12.01.2022
(62) Divisional of application: 15724438.5
(73) Proprietor: Neurocrine Biosciences, Inc., San Diego, California 92130 (US)
(72) Inventor: O'BRIEN, Christopher, San Diego, 92130 (US)
(74) Representative: Mewburn Ellis LLP

(56) References cited:
- WO-A1-2008/058261
- WO-A1-2014/047167
- WO-A1-2015/120317
- WO-A2-2011/019956
- WO-A2-2011/153157
- US-A1- 2011 053 866
- Anonymous: "Neurocrine Announces Phase IIb Results Of VMAT2 Inhibitor NBI-98854 For Treatment Of Tardive Dyskinesia", Neurocrine Biosciences : Investors : Press Release, 9 September 2013 (2013-09-09), XP055197770, Retrieved from the Internet: URL:http://phoenix.corporate-ir.net/phoeni x.zhtml?c=68817&p=irol-newsArticle_Print&I D=1853185 [retrieved on 2015-06-23]
- SIEGERT SCOTT ET AL: "Efficacy and Safety of Valbenazine (NBI-98854) in Subjects with Tardive Dyskinesia: Results of a Long-Term Study (KINECT 3 Extension)", THE XXIITH WORLD CONGRESS ON PARKINSON'S DISEASE AND RELATED DISORDERS, 12 November 2017 (2017-11-12), XP55803088,

## Description

### BACKGROUND

### Technical Field

Described herein are methods for obtaining an optimum treatment of hyperkinetic movement disorders in a subject wherein efficacious blood plasma concentrations of (+)α-3-isobutyl-9,10-dimethoxy-1,3,4,6,7,1 1b-hexahydro-2H-pyrido[2,1-a]isoquinolin-2-ol ((+)α-HTBZ) are achieved.

### Description of the Related Art

Dysregulation of dopaminergic systems is integral to several central nervous system (CNS) disorders, including hyperkinetic movement disorders (e.g., tardive dyskinesia (TD)) and conditions such as schizophrenia and bipolar disorder. The transporter protein vesicular monoamine transporter 2 (VMAT2) plays an important role in presynaptic dopamine release, regulating monoamine uptake from the cytoplasm to the synaptic vesicle for storage and release. The differential expression of VMAT2 in human brain versus endocrine tissue provides an opportunity for use of a well-tolerated agent that selectively targets VMAT2 potentially useful for the treatment of CNS disorders *(see, e.g.,* Weihe and Eiden, The FASEB Journal 2000, 14: 2345-2449).

Tardive dyskinesia (TD) is a neurological condition characterized by involuntary movements of the orofacial region *(i.e.,* tongue, lips, jaw, face) and choreoathetoid movements in the limbs and trunk. Mild symptoms of TD are usually not treated. Patients with mild TD are typically unaware of the involuntary movements and they do not seek treatment. As symptom severity increases, the hyperkinetic movements begin to disrupt normal speech, chewing, breathing, facial expression, limb movements, walking and balance. At this point the potential benefit of pharmacological treatment outweighs the potential risk of treatment-related side effects. In the most severe cases, TD may result in self-injury, abrasions, lacerations, inability to dress, eat, or drink. For a recent overview of the tardive syndromes, see Bhidayasiri and Boonyawairoj, Postgrad Med J 2011, 87(1024): 132-141.

TD develops with long-term neuroleptic drug use and often persists after discontinuation of the offending medication. A small proportion of patients who are treated with dopamine receptor blocking drugs develop TD; most often these patients have schizophrenia. While the pathophysiology of TD is not fully understood, postsynaptic dopamine hypersensitivity in the striatum is the most prominent feature. TD is differentiated from the acute signs and symptoms of dopamine blockade such as akathisia or parkinsonism. These acute exposure symptoms are often described as "extra-pyramidal side effects" or EPSE, not a tardive or delayed response. While isolated case reports of TD after short-term exposure exist, most often TD emerges after long-term treatment over months to years. In addition to duration and amount of neuroleptic exposure, other risk factors for TD appear to include older age, schizophrenia and cognitive impairment (Margolese et al., Can J Psychiatry 2005, 50 (9): 541-47).

Most recent literature regarding TD suggests a prevalence rate of TD in approximately 15% of psychiatric patients with extremely low rates in other non-psychiatric populations *(see, e.g.,* Tarsy and Baldessarini, Movement Disorders 2006, 21(5): 589-98). This clustering of TD in schizophrenia patients likely reflects that TD usually emerges in the setting of chronic exposure. In contrast, short-term use of dopamine antagonists is rarely associated with TD. Most reviews of TD describe, on average, greater than five years exposure to these agents. The DSM-IV and the clinical research criteria for TD (*e.g.,* the Schooler-Kane Criteria of 1982) note a requirement that exposure be documented for greater than three months to confirm the diagnosis. Recent assessments indicate that the incidence of tardive hyperkinetic movements in patients receiving chronic neuroleptic medications is approximately 1-5% per year of exposure *(see, e.g.,* Tenback et al, J Psychopharmacol 2010, 24: 1031; Woods et al, J Clin Psychiatry 2010, 71(4): 463-74). Remission is reported in 30-60% of patients who no longer take the offending agent for several years.

The incidence of TD was hoped to be substantially lower following the availability of the atypical or so-called second generation antipsychotic medications. However, a decrease in incidence has been only partially borne out by the literature. Short-term trials of 12 months or less report very little TD, whereas the longer, nonindustry sponsored trials suggest the prevalence is closer to 4-6% *(see, e.g.,* Correll and Schenk, Curr Opin Psychiatry 2008, 21(2): 151-6). Patients with bipolar disorder (BD) are also prescribed antipsychotic medications, particularly if refractory to first line medications. Second-generation atypical antipsychotics are commonly prescribed for BD treatment. Alternative therapies for BD are available (*e.g*., lithium, valproate, etc.); therefore, patients with emerging signs of TD are often able to discontinue exposure to the offending agent and continue their standard treatment, which may allow for remission of TD.

TD may develop in patients with non-psychiatric disorders, who are treated with limited-duration and occasionally longer-duration exposure to dopamine receptor antagonists (*e.g*., REGLAN^{®} [metoclopramide] for gastroparesis). While the awareness of REGLAN^{®} associated side effects has increased and become the focus of class-action law suits, metoclopramide-induced TD appears to actually occur in <1% of patients exposed to drug *(see, e.g.,* Rao et al, Aliment Pharmacol Ther 2010, 31(1): 11).

Neither a standard treatment regimen nor an approved drug is available for treatment of TD. The first step to treating this condition is generally to stop or minimize the use of the neuroleptic drug suspected of causing the condition. Replacing the offending drug with an alternative antipsychotic drug, such as clozapine, may help some patients. Some experimental work has been conducted with more aggressive interventions such as Deep Brain Stimulation for severe cases. In addition, vesicular monoamine transporter 2 (VMAT2) inhibitors have been shown effective in treatment of various movement disorders, including tardive dyskinesia *(see, e.g.,* Ondo et al, Am J Psychiatry 1999, 156(8): 1279-1281; Jankovic and Beach, Neurology 1997, 48: 359-362). A well tolerated oral medication for patients with moderate or severe TD could provide an important treatment option for this condition. In a Press Release dated 09.09.2013, Neurocrine Biosciences Inc. announced Phase IIb results of NBI-98854 for the treatment of tardive dyskinesia, stating that a 50mg dose did not meet the primary end-point in the Phase IIb Kinect study, whilst the 100mg dose showed statistical and clinically-significant improvement. It went on to state that a daily dose of up to 75mg was under investigation in the Kinect 2 study. Therefore, a need in the art exists for a therapy useful for treating TD.

### BRIEF SUMMARY

The invention provides a pharmaceutical composition comprising the VMAT2 inhibitor (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof, for use in a method of treating a hyperkinetic movement disorder in a human subject, said method comprising administering to the human subject the VMAT2 inhibitor (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester or pharmaceutically acceptable salt thereof at a daily dose of 80mg

These and other embodiments within the scope of the appended claims will be apparent upon reference to the following detailed description. The invention is defined by the claims.

Terms not specifically defined herein should be given the meanings that would be given to them by one of skill in the art in light of the disclosure and the context. As used in the specification, however, unless specified to the contrary, the terms have the meaning indicated.

Reference throughout this specification to "one embodiment" or "an embodiment" means that a particular feature, structure or characteristic described in connection with the embodiment is included in at least one embodiment. Thus, the appearances of the phrases "in one embodiment" or "in an embodiment" in various places throughout this specification are not necessarily all referring to the same embodiment. Furthermore, the particular features, structures, or characteristics may be combined in any suitable manner in one or more embodiments.

Also, as used in this specification and the appended claims, the singular forms "a," "an," and "the" include plural referents unless the content clearly dictates otherwise. Thus, for example, reference to "a non-human animal" may refer to one or more non-human animals, or a plurality of such animals, and reference to "a cell" or "the cell" includes reference to one or more cells and equivalents thereof (*e.g*., plurality of cells) known to those skilled in the art, and so forth. When steps of a method are described or claimed, and the steps are described as occurring in a particular order, the description of a first step occurring (or being performed) "prior to" (*i.e.,* before) a second step has the same meaning if rewritten to state that the second step occurs (or is performed) "subsequent" to the first step. The term "about" when referring to a number or a numerical range means that the number or numerical range referred to is an approximation within experimental variability (or within statistical experimental error), and thus the number or numerical range may vary between 1% and 15% of the stated number or numerical range. It should also be noted that the term "or" is generally employed in its sense including "and/or" unless the content clearly dictates otherwise. The term, "at least one," for example, when referring to at least one compound or to at least one composition, has the same meaning and understanding as the term, "one or more."

### BRIEF DESCRIPTION OF THE DRAWING

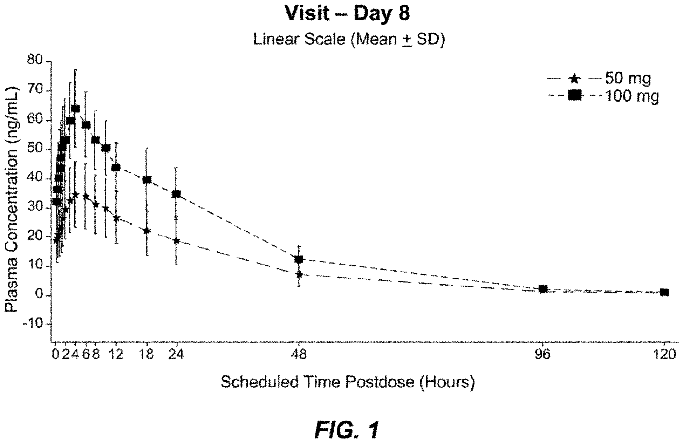

Figure 1 shows mean plasma concentration data (linear scale) of active metabolite (+)α-HTBZ vs. scheduled time post-dose on day 8 for patients in NBI-98854 multiple dose cohort study.

### DETAILED DESCRIPTION

Described herein are methods for attaining or maintaining an optimal concentration of at least one active metabolite of tetrabenazine (3-isobutyl-9,10-dimethoxy-1,3,4,6,7,1 1b-hexahydro-2H-pyrido[2,1-a]isoquinolin-2-one, TBZ) in a subject by administering to a subject the compound TBZ at a dose of the compound that attains or maintains a concentration of an active metabolite (*e.g*., (+)α-HTBZ) over a specified period of time, thereby optimizing clinical benefit for treating hyperkinetic movement disorders (*e.g*., TD). Prior to the disclosure herein, doses of TBZ or analogs thereof that resulted in optimal clinical benefit to a subject appeared to vary for the individual treated subjects.

Tetrabenazine (XENAZINE^{®}) is an approved agent with VMAT2 inhibitory activity, a dopamine-depleting agent approved in 2008 for the treatment of chorea associated with Huntington's disease. However, XENAZINE^{®} is not approved for TD, and tetrabenazine has a strict Risk Evaluation and Mitigation Strategy (REMS) program limiting distribution to Huntington's disease patients only. Clinical benefit has been described with tetrabenazine when used under Physician IND for the treatment of TD and a variety of hyperkinetic movement disorders *(see, e.g.,* Ondo et al, Am J Psychiatry 1999, 156(8): 1279-1281; Jankovic and Beach, Neurology 1997, 48: 359-362). The beneficial pharmacologic effects of tetrabenazine on the targeted hyperkinetic involuntary movements have been documented, as well as the adverse effects associated with excessive monoamine depletion, such as sedation, depression, akathisia and parkinsonism. The occurrence of these adverse effects with tetrabenazine has resulted in the need for individualized dosing, dose titration, and management of treatment-related side effects under a restrictive REMS program.

The requirement for dose titration with tetrabenazine in the clinic may be due to its extensive and variable metabolism. TBZ, which contains two chiral centers and is a racemic mix of two stereoisomers, is rapidly and extensively metabolized *in vivo* to its reduced form, 3-isobutyl-9,10-dimethoxy-1,3,4,6,7,11b-hexahydro-2H-pyrido[2,1-a]isoquinolin-2-ol, also known as dihydrotetrabenazine (HTBZ). HTBZ is thought to exist as four individual isomers: (±)α-HTBZ and (±) beta-HTBZ. The (2R, *3R,* 11b*R*)-3-isobutyl-9,10-dimethoxy-1,3,4,6,7,11b-hexahydro-2H-pyrido[2,1-a]isoquinolin-2-ol or (+)α-HTBZ is believed to be the absolute configuration of the most active metabolite *(see, e.g.,* Kilbourn Chirality 1997 9:59-62). Since tetrabenazine is rapidly metabolized, and since very low exposures of tetrabenazine are observed upon its oral administration, the therapeutic efficacy of tetrabenazine appears to be due primarily to actions of metabolites (+)α-HTBZ and (+)β-HTBZ *(see, e.g.,* Kilbourn et al., Eur J Pharmacol 1995, 278: 249-252; Mehvar et al., Drug Metabolism and Disposition 1987, 15(2): 250-255; Xenazine Package Insert, Biovail Laboratories International, 2009). Metabolism of tetrabenazine to (±)α-HTBZ and (±)β-HTBZ is highly variable between patients *(see, e.g.,* Mehvar et al., Drug Metabolism and Disposition 1987, 15(2): 250-255). Additionally, these stereoisomers of HTBZ exhibit varying pharmacology *(i.e.,* binding to off-target protein receptors) *(see, e.g.,* Kilbourn et al., Eur J Pharmacol 1995, 278: 249-252). This represents a source of added risk to the patient and complication for the physician in terms of actively managing a patient's dosing regimen.

Described herein are methods for treating a subject who has a hyperkinetic movement disorder with [+]α-dihydrotetrabenazine or a precursor thereof in a sufficient amount to achieve an appropriate concentration over a specified period of time of [+]α-dihydrotetrabenazine in plasma.

In one embodiment, a pharmaceutical composition comprising the VMAT2 inhibitor (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof, for use in a method of treating a hyperkinetic movement disorder in a human subject in need thereof, said method comprising administering to the human subject the VMAT2 inhibitor, or the pharmaceutically acceptable salt thereof, at a daily dosage of 80 mg is disclosed.

Reference to plasma concentration of (+)α-HTBZ in the methods described herein includes both deuterated (+)α-HTBZ and non-deuterated (+)α-HTBZ. It is apparent to a person of skill in the art that if a deuterated VMAT2 inhibitor as described herein is administered to a subject (*e.g*., deuterated TBZ, deuterated (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or deuterated (+)α-HTBZ), then deuterated (+)α-HTBZ will appear in the subject's blood plasma and is to be measured. If a non-deuterated VMAT2 inhibitor as described herein is administered to a subject (*e.g.,* TBZ, (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, (+)α-HTBZ), then non-deuterated (+)α-HTBZ will appear in the subject's blood plasma and is to be measured. If a combination of deuterated and non-deuterated VMAT2 inhibitors as described herein is administered to a subject, then both deuterated and non-deuterated (+)α-HTBZ will appear in the subject's blood plasma and both are to be measured.

In certain methods described herein, the Cₘₐₓ of (+)α-HTBZ is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma. In certain cases, the Cₘᵢₙ of (+)α-HTBZ is at least 15 ng/mL, at least 20 ng/mL, at least 25 ng/mL, at least 30 ng/mL, or at least 35 ng/mL plasma, over a period of 8 hrs, 12 hrs, 16 hrs, 20 hrs, 24 hrs, 28 hrs, or 32 hrs. In certain cases, the Cₘᵢₙ of (+)α-HTBZ is between about 15 ng/mL to about 35 ng/mL.

In certain aspects, the VMAT2 inhibitor for use described herein achieves a maximal blood plasma concentration (Cₘₐₓ) of (+)β-3-isobutyl-9,10-dimethoxy-1,3,4,6,7,1 1b-hexahydro-2H-pyrido[2,1-a]isoquinolin-2-ol ((+)β-HTBZ) of between about 15 ng to about 60 ng per mL plasma and a minimal blood plasma concentration (Cₘᵢₙ) of (+)β-3-isobutyl-9,10-dimethoxy-1,3,4,6,7,11b-hexahydro-2H-pyrido[2,1-a]isoquinolin-2-ol ((+)β -HTBZ) of at least 15 ng per mL plasma over an 8 hour period.

In certain aspects, the Cₘₐₓ of (+)β-HTBZ is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma. In certain embodiments, the Cₘᵢₙ of (+)P-HTBZ is at least 15 ng/mL, at least 20 ng/mL, at least 25 ng/mL, at least 30 ng/mL, or at least 35 ng/mL plasma, over a period of 8 hrs, 12 hrs, 16 hrs, 20 hrs, 24 hrs, 28 hrs, or 32 hrs. In certain embodiments, the Cₘᵢₙ of (+)β-HTBZ is about 15 ng/mL to about 35 ng/mL.

In certain aspects, the therapeutic concentration range is about 15 ng to about 35 ng, to about 40 ng, to about 45 ng, to about 50 ng, or to about 55 ng (+)α-3-isobutyl-9,10-dimethoxy-1,3,4,6,7,11b-hexahydro-2H-pyrido[2,1-a]isoquinolin-2-ol ((+)α-HTBZ)per mL plasma.

In certain aspects, the threshold concentration of (+)α-HTBZ is about 15 ng/mL, about 20 ng/mL, about 25 ng/mL, about 30 ng/mL, about 35 ng/mL, about 40 ng/mL, about 45 ng/mL, about 50 ng/mL, about 55 ng/mL or about 60 ng/mL plasma, over a period of about 8 hrs, about 12 hrs, about 16 hrs, about 20 hrs, about 24 hrs, about 28 hrs, or about 32 hrs. In a particular embodiment, the threshold concentration of (+)α-HTBZ is between about 15 ng/mL to about 35 ng/mL over a period of about 8 hours to about 24 hours.

In a specific embodiment, the pharmaceutical composition for use in the methods described herein comprises (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester. In a more specific embodiment, the compound is the dihydrochloride or ditosylate salt.

In a specific embodiment, the composition is for administration to a subject in a daily dose of 80 mg of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester.

Plasma concentrations of (+)α-HTBZ, (+)β-HTBZ, and compounds as disclosed herein may be measured by methods as described in Derangula et al, Biomedical Chromatography 2013 27(6): 792-801, Mehvar et al, Drug Metabolism and Distribution 1987 15(2): 250-55 and generally by tandem mass spectroscopy.

As discussed herein, the compounds described herein and their salts may reduce the supply of monoamines in the central nervous system by inhibiting the human monoamine transporter isoform 2 (VMAT2). As such, these compounds and their salts may have utility over a wide range of therapeutic applications, and may be used to treat a variety of disorders which are caused by or linked to inhibition of the human monoamine transporter isoform 2. These disorders include hyperkinetic disorders. Conditions which may be treated by compounds described herein include, but are not limited to, hyperkinetic disorders such as Huntington's disease, tardive dyskinesia, Tourette syndrome, dystonia, hemiballismus, chorea, and tics.

The compounds described herein may be synthesized according to known organic synthesis techniques described in the art. See for example, U.S. Patent No. 8,039,627 that describes general synthesis schemes and methods for synthesis of specific compounds including (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester (called 2-1 in U.S. Patent No. 8,039,627).

The compounds described herein may generally be used as the free acid or free base. Alternatively, the compounds may be used in the form of acid or base addition salts. Acid addition salts of the free amino compounds may be prepared by methods well known in the art, and may be formed from organic and inorganic acids. Suitable organic acids include maleic, fumaric, benzoic, ascorbic, succinic, methanesulfonic, acetic, trifluoroacetic, oxalic, propionic, tartaric, salicylic, citric, gluconic, lactic, mandelic, cinnamic, aspartic, stearic, palmitic, glycolic, glutamic, and benzenesulfonic acids. Suitable inorganic acids include hydrochloric, hydrobromic, sulfuric, phosphoric, and nitric acids. Base addition salts included those salts that form with the carboxylate anion and include salts formed with organic and inorganic cations such as those chosen from the alkali and alkaline earth metals (for example, lithium, sodium, potassium, magnesium, barium and calcium), as well as the ammonium ion and substituted derivatives thereof (for example, dibenzylammonium, benzylammonium, 2-hydroxyethylammonium, and the like). Thus, the term "pharmaceutically acceptable salt" of the compounds described herein is intended to encompass any and all acceptable salt forms.

With regard to stereoisomers, the compounds described herein may have chiral centers and may occur as racemates, racemic mixtures and as individual enantiomers or diastereomers. All such isomeric forms are included within the present invention, including mixtures thereof. Furthermore, some of the crystalline forms of the compounds may exist as polymorphs, which are contemplated herein. In addition, some of the compounds may also form solvates with water or other organic solvents. Such solvates are similarly included within the scope of the compounds described herein.

As one of skill in the art would appreciate, any of the aforementioned compounds may incorporate radioactive isotopes. Accordingly, also contemplated is use of isotopically-labeled compounds identical to those described herein, wherein one or more atoms are replaced by an atom having an atomic mass or mass number different from the atomic mass or mass number usually found in nature. Examples of isotopes that can be incorporated into these compounds include isotopes of hydrogen, carbon, nitrogen, oxygen, phosphorous, fluorine and chlorine, such as, but not limited to, ²H, . ³H, ¹³C, ¹⁴C, ¹⁵N, ¹⁸O, ¹⁷O, ³¹P, ³²P, ³⁵S, ¹⁸F, and ³⁶Cl, respectively. Certain isotopically-labeled compounds, for example those into which radioactive isotopes such as ³H and ¹⁴C are incorporated, are also useful in drug or substrate tissue distribution assays. Tritiated hydrogen (³H) and carbon-14 (¹⁴C) isotopes are particularly preferred for their ease of preparation and detectability. Substitution with heavier isotopes such as deuterium (²H) can provide certain therapeutic advantages resulting from greater metabolic stability, for example increased in vivo half-life or reduced dose requirements and, therefore, may be preferred in some circumstances. Isotopically-labeled compounds can generally be prepared by performing procedures routinely practiced in the art.

NBI-98854 ((S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester), and pharmaceutically acceptable salts thereof, is an orally active valine ester of vesicular monoamine transporter 2 (VMAT2) inhibitor ((+) alpha-HTBZ) and was designed to deliver [+]α-HTBZ in a controlled fashion with reduced peak plasma concentrations and pharmacokinetic (PK) variability that is intended to limit off-target binding and to allow for an improved safety profile in human subjects.

The selective targeting of VMAT2 and the pharmacokinetic profile of the active metabolite [+]α-HTBZ appeared to provide both preferential and a doserelated modulation of dopamine release without evidence of significant serotonin, norepinephrine, or histamine depletion. This should be possible when administered either in conjunction with a patient's existing antipsychotic medication or independently in patients for whom the antipsychotic medication has been discontinued.

As understood by a person skilled in the medical art, the terms, "treat" and "treatment," refer to medical management of a disease, disorder, or condition of a subject (*i.e*., patient) *(see, e.g.,* Stedman's Medical Dictionary). The terms "treatment" and "treating" embraces both preventative, i.e. prophylactic, or therapeutic, i.e. curative and/or palliative, treatment. Thus the terms "treatment" and "treating" comprise therapeutic treatment of patients having already developed the condition, in particular in manifest form. Therapeutic treatment may be symptomatic treatment in order to relieve the symptoms of the specific indication or causal treatment in order to reverse or partially reverse the conditions of the indication or to stop or slow down progression of the disease. Thus the compositions and methods described herein may be used, for instance, as therapeutic treatment over a period of time as well as for chronic therapy. In addition the terms "treatment" and "treating" comprise prophylactic treatment, *i.e.,* a treatment of patients at risk to develop a condition mentioned hereinbefore, thus reducing the risk.

The human subject in need of the compositions and methods described herein includes a human subject who has been diagnosed by a person skilled in the medical and psychiatric arts with a hyperkinetic disorder (*e.g*., tardive dyskinesia).

Therapeutic and/or prophylactic benefit includes, for example, an improved clinical outcome, both therapeutic treatment and prophylactic or preventative measures, wherein the object is to prevent or slow or retard (lessen) an undesired physiological change or disorder, or to prevent or slow or retard (lessen) the expansion or severity of such disorder. Prophylactic administration of a composition herein may commence upon first treatment with dopamine receptor blocking drugs such as neuroleptics. As discussed herein, beneficial or desired clinical results from treating a subject include, but are not limited to, abatement, lessening, or alleviation of symptoms that result from or are associated the disease, condition, or disorder to be treated; decreased occurrence of symptoms; improved quality of life; longer disease-free status *(i.e.,* decreasing the likelihood or the propensity that a subject will present symptoms on the basis of which a diagnosis of a disease is made); diminishment of extent of disease; stabilized *(i.e.,* not worsening) state of disease; delay or slowing of disease progression; amelioration or palliation of the disease state; and remission (whether partial or total), whether detectable or undetectable; and/or overall survival. "Treatment" can also mean prolonging survival when compared to expected survival if a subject were not receiving treatment. Subjects in need of treatment include those who already have the condition or disorder as well as subjects prone to have or at risk of developing the disease, condition, or disorder (*e.g*., TD or other conditions or disorders described herein), and those in which the disease, condition, or disorder is to be prevented (*i.e*., decreasing the likelihood of occurrence of the disease, disorder, or condition). A therapeutically effective amount of any one of the compounds described herein in the amount of the compound that provides a statistically or clinically significant therapeutic and/or prophylactic benefit to the treated subject.

Methods for determining the effectiveness of a therapeutic for treating a hyperkinetic disorder are routinely practiced in the art by a person skilled in the medical and clinical arts. By way of example, a subject with a hyperkinetic disorder may be diagnosed, monitored, and evaluated by the Abnormal Involuntary Movement Scale (AIMS). The AIMS is a structured neurological examination that was developed in 1976 and has been used extensively in movement disorder assessments. It consists of seven distinct ratings of regional involuntary body movements that are scored on a zero to four scale with zero being rated as none and four being rated as severe.

### Pharmaceutical Compositions

The present disclosure provides a pharmaceutical composition comprising the VMAT2 inhibitor (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof, for use in a method of treating a hyperkinetic movement disorder in a human subject in need thereof, said method comprising administering to the human subject the VMAT2 inhibitor, or the pharmaceutically acceptable salt thereof, at a daily dosage of 80 mg. A pharmaceutically acceptable excipient is a physiologically and pharmaceutically suitable non-toxic and inactive material or ingredient that does not interfere with the activity of the active ingredient; an excipient also may be called a carrier. The formulation methods and excipients described herein are exemplary and are in no way limiting. Pharmaceutically acceptable excipients are well known in the pharmaceutical art and described, for example, in Rowe et al., Handbook of Pharmaceutical Excipients: A Comprehensive Guide to Uses, Properties, and Safety, 5th Ed., 2006, and in Remington: The Science and Practice of Pharmacy (Gennaro, 21st Ed. Mack Pub. Co., Easton, PA (2005)). Exemplary pharmaceutically acceptable excipients include sterile saline and phosphate buffered saline at physiological pH. Preservatives, stabilizers, dyes, buffers, and the like may be provided in the pharmaceutical composition. In addition, antioxidants and suspending agents may also be used.

For compositions formulated as liquid solutions, acceptable carriers and/or diluents include saline and sterile water, and may optionally include antioxidants, buffers, bacteriostats and other common additives. The compositions can also be formulated as pills, capsules, granules, or tablets which contain, in addition to a VMAT2 inhibitor, diluents, dispersing and surface active agents, binders, and lubricants. One skilled in this art may further formulate the VMAT2 inhibitor in an appropriate manner, and in accordance with accepted practices, such as those disclosed in *Remington, supra.*

As used herein, systemic administration includes oral and parenteral methods of administration. For oral administration, suitable pharmaceutical compositions include powders, granules, pills, tablets, and capsules as well as liquids, syrups, suspensions, and emulsions. These compositions may also include flavorants, preservatives, suspending, thickening and emulsifying agents, and other pharmaceutically acceptable additives. For parental administration, the compounds of the present invention can be prepared in aqueous injection solutions which may contain, in addition to the VMAT2 inhibitor, buffers, antioxidants, bacteriostats, and other additives commonly employed in such solutions.

As described herein optimal doses are generally determined using experimental models and/or clinical trials. The optimal dose may depend upon the body mass, weight, blood volume, or other individual characteristics of the subject. For example, a person skilled in the medical art can consider the subject's condition, that is, stage of the disease, severity of symptoms caused by the disease, general health status, as well as age, gender, and weight, and other factors apparent to a person skilled in the medical art. Subjects may generally be monitored for therapeutic effectiveness by clinical evaluation and using assays suitable for the condition being treated or prevented, which methods (*e.g*., AIMS evaluation) will be familiar to those having ordinary skill in the art and are described herein. The concentration of a compound that is administered to a subject may be monitored by determining the concentration of the compound in a biological fluid, for example, in the blood, blood fraction (*e.g*., plasma, serum), and/or in the urine, and/or other biological sample from the subject. Any method practiced in the art to detect the compound may be used to measure the concentration of compound during the course of a therapeutic regimen.

Extended release formulations of tetrabenazine and d₆-tetrabenazine are known in the art. Extended release pharmaceutical compositions have been described in PCT Publications WO 2010/018408, WO 2011/019956 and WO2014/047167.

The pharmaceutical compositions described herein that comprise the VMAT2 inhibitor described herein may be administered to a subject in need by any one of several routes that effectively deliver an effective amount of the compound. Such administrative routes include, for example, oral, parenteral, enteral, rectal, intranasal, buccal, sublingual, intramuscular, and transdermal.

### EXAMPLES

### EXAMPLE 1

### HUMAN CLINICAL TRIALS - NBI-98854

Clinical data from TD subjects administered repeated doses of (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester from 12.5 mg to 100 mg per day indicates the drug is generally well tolerated. Efficacy is related to the concentrations of the active metabolite [+]α-dihydrotetrabenazine. Exposure-response analysis indicates that a concentration of 30 ng/mL in plasma is an appropriate target. Exposures above 60 ng/mL in plasma afford little incremental benefit but increase the risk of adverse events reflecting extension of VMAT2 pharmacology. Exposures below 15 ng/mL are suboptimal across the general TD population.

Observed exposure and Abnormal Involuntary Movement Scale (AIMS) derived from video ratings from a Phase 2 clinical study of (S)-2-amino-3-methylbutyric acid (2R,3R,11bR)-3-isobutyl-9,10-dimethoxy-1,3,4,6,7,1 1b-hexahydro-2H-pyrido[2,1-a]isoquinolin-2-yl ester were employed for development of the exposure-response relationship. A total of 96 patients were randomized to placebo (N=41) and (S)-2-amino-3-methyl-butyric acid (2R,3R,11bR)-3-isobutyl-9,10-dimethoxy-1,3,4,6,7,1 1b-hexahydro-2H-pyrido[2,1-a]isoquinolin-2-yl ester (either 25mg, 50 mg or 75mg, N=45). Thirty-eight patients receiving (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester had PK data available for the metabolite [+]α-dihydrotetrabenazine. Hence data from a total of 79 (41 placebo and 38 drug exposed) patients were used in the exposure-AIMS analysis. AIMS derived from video rating was available at baseline and week 6 in each patient. Percent change from baseline at week 6 served as the response metric. [+]α-Dihydrotetrabenazine concentration at week 6 sampled at about the Tₘₐₓ was available in each patient and this Cₘₐₓ steady state (ss) was used as the exposure measure.

The patients who had quantifiable concentrations of [+]α-dihydrotetrabenazine were divided into quartiles based upon the week 6 Cₘₐₓ ss. The mean Cₘₐₓ ss and mean AIMS reduction from baseline were calculated for each quartile. These quartiles were compared to placebo patients as shown in the following table.

| | **Mean Plasma concentration** (Cmax ss, ng/mL of [+]α-HTBZ) | **Mean AIMS reduction - %** (week 6 vs baseline) |
|---|---|---|
| Placebo | 0 | 2 |
| 1^{st} quartile | 15 | 33 |
| 2^{nd} quartile | 29 | 70 |
| 3^{rd} quartile | 42 | 65 |
| 4^{th} quartile | 93 | 52 |

As can be seen, the placebo patients' AIMS at the week 6 time point was substantially similar to the baseline reading. The first quartile of patients (those patients who showed the lowest Cmax ss of [+]α-HTBZ at the 6 week time point regardless of the dose of NBI-98854 administered) showed a mean Cmax ss of [+]α-HTBZ of approximately 15 ng/mL and a modest reduction in AIMS of approximately 33%. The next quartile of patients showed a mean Cmax ss of [+]α-HTBZ of approximately 29 ng/mL and a maximal reduction in AIMS of approximately 70% from baseline reading. The patients achieving the highest concentrations of [+]α-HTBZ in the next 2 quartiles did not achieve any greater reduction in TD symptoms as measured by reduction in mean AIMS score versus the 2^{nd} quartile patients.

### EXAMPLE 2

### MAINTENANCE OF PLASMA THRESHOLD CONCENTRATION OF [+]α,-HTBZ IN NBI-98854 TREATED PATIENTS

NBI-98854 was administered orally once daily at a dose of 50 mg or 100 mg for 8 days to patients in a multiple-dose cohort (n=13 for 50 mg dosage group; n=4 for 100 mg dosage group). Individual subject plasma concentration data for (+)α-HTBZ was collected at scheduled times post-dose (0 hr, 2 hr, 4 hr, 6 hr, 8 hr, 12 hr, 16 hr, 24 hr, 48 hr, 96 hr, and 120 hr) on day 8 and presented as mean plasma concentration data (linear scale) (*see,* Figure 1). On Day 8, median time to Tₘₐₓ for (+)α-HTBZ was approximately 4.0 hours for both doses. After maximal concentration (Cₘₐₓ) was attained, (+)α-HTBZ plasma concentrations appeared to decline and exhibited an apparent t_{1/2} of approximately 21 hours (50 mg dose) and approximately 19 hours (100 mg dose). As shown in Figure 1, the 50 mg dose of NBI-98854 appeared to maintain a desired therapeutic concentration range of between about 15 to about 60 ng of (+)α-HTBZ per mL plasma, above a threshold concentration of about 15 ng/mL over a period of about 8 to about 24 hours.

## Claims

1. A pharmaceutical composition comprising the VMAT2 inhibitor (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester, or a pharmaceutically acceptable salt thereof, for use in a method of treating a hyperkinetic movement disorder in a human subject in need thereof, said method comprising administering to the human subject the VMAT2 inhibitor, or the pharmaceutically acceptable salt thereof, at a daily dosage of 80 mg.

2. The pharmaceutical composition for use according to claim 1, wherein the hyperkinetic movement disorder is tardive dyskinesia.

3. The pharmaceutical composition for use according to claim 1, wherein hyperkinetic movement disorder is selected from the group consisting of Huntington's disease, tardive dyskinesia, Tourette syndrome, dystonia, hemiballismus, chorea, and tics.

4. The pharmaceutical composition for use according to any one of claims 1 to 3, wherein the pharmaceutical composition comprises an extended release formulation of the VMAT2 inhibitor.

5. The pharmaceutical composition for use according to any one of claims 1 to 4, wherein the VMAT2 inhibitor is (S)-2-amino-3-methyl-butyric acid (2R,3R,11bR)-3-isobutyl-9,10-dimethoxy-1,3,4,6,7,11b-hexahydro-2H-pyrido[2,1-a]isoquinolin-2-yl ester.

6. The pharmaceutical composition for use according to any one of claims 1 to 5, wherein the pharmaceutically acceptable salt is the dihydrochloride salt or ditosylate salt.

7. The pharmaceutical composition for use according to any one of claims 1 to 6 wherein the composition takes the form of a powder, granule, pill, tablet, capsule, liquid, syrup, suspension or emulsion suitable for oral administration.

8. The pharmaceutical composition for use according to any one of claims 1 to 6, wherein the composition is administered to the human subject in need thereof via the oral, parenteral, enteral, rectal, intranasal, buccal, sublingual, intramuscular or transdermal route.

## Patentansprüche

1. Pharmazeutische Zusammensetzung, umfassend den VMAT2-Hemmer (S)-2-Amino-3-methylbuttersäure-(2R,3R,11bR)-3-isobutyl-9,10-dimethoxy-1,3,4,6,7,11b-hexahydro-2H-pyrido[2,1-a]isochinolin-2-ylester oder ein pharmazeutisch annehmbares Salz davon, zur Verwendung in einem Verfahren zur Behandlung einer hyperkinetischen Bewegungsstörung bei einem menschlichen Individuum mit Bedarf daran, wobei das Verfahren das Verabreichen des VMAT2-Hemmers oder des pharmazeutisch annehmbaren Salzes davon an das menschliche Individuum bei einer täglichen Dosierung von 80 mg umfasst.

2. Pharmazeutische Zusammensetzung zur Verwendung nach Anspruch 1, wobei es sich bei der hyperkinetischen Bewegungsstörung um tardive Dyskinesie handelt.

3. Pharmazeutische Zusammensetzung zur Verwendung nach Anspruch 1, wobei die hyperkinetische Bewegungsstörung aus der aus Huntington-Krankheit, tardiver Dyskinesie, Tourette-Syndrom, Dystonie, Hemiballismus, Chorea und Tics bestehenden Gruppe ausgewählt ist.

4. Pharmazeutische Zusammensetzung zur Verwendung nach einem der Ansprüche 1 bis 3, wobei die pharmazeutische Zusammensetzung eine Formulierung mit verzögerter Freisetzung des VMAT2-Hemmers umfasst.

5. Pharmazeutische Zusammensetzung zur Verwendung nach einem der Ansprüche 1 bis 4, wobei der VMAT2-Hemmer (S)-2-Amino-3-methylbuttersäure-(2R,3R,11bR)-3-isobutyl-9,10-dimethoxy-1,3,4,6,7,11b-hexahydro-2H-pyrido[2,1-a]isochinolin-2-ylester ist.

6. Pharmazeutische Zusammensetzung zur Verwendung nach einem der Ansprüche 1 bis 5, wobei das pharmazeutisch annehmbare Salz Dihydrochloridsalz oder Ditosylatsalz ist.

7. Pharmazeutische Zusammensetzung zur Verwendung nach einem der Ansprüche 1 bis 6, wobei die Zusammensetzung in Form eines Pulvers, eines Granulats, einer Pille, einer Tablette, einer Kapsel, einer Flüssigkeit, eines Sirups, einer Suspension oder einer Emulsion, der/die/das für die orale Verabreichung geeignet ist, vorliegt.

8. Pharmazeutische Zusammensetzung zur Verwendung nach einem der Ansprüche 1 bis 6, wobei die Zusammensetzung dem menschlichen Individuum mit Bedarf daran auf oralem, parenteralem, enteralem, rektalem, intranasalem, bukkalem, sublingualem, intramuskulärem oder transdermalem Weg verabreicht wird.

## Revendications

1. Composition pharmaceutique comprenant l'inhibiteur de de VMAT2 d'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthyl-butyrique, ou un sel pharmaceutiquement acceptable de celui-ci, à utiliser dans un procédé de traitement d'un trouble du mouvement hypercinétique chez un sujet humain qui en a besoin, ledit procédé comprenant l'administration au sujet humain de l'inhibiteur de VMAT2, ou du sel pharmaceutiquement acceptable de celui-ci, à une dose quotidienne de 80 mg.

2. Composition pharmaceutique à utiliser selon la revendication 1, dans laquelle le trouble du mouvement hyperkinétique est une dyskinésie tardive.

3. Composition pharmaceutique à utiliser selon la revendication 1, dans laquelle le trouble du mouvement hyperkinétique est choisi dans le groupe constitué de la maladie de Huntington, de la dyskinésie tardive, du syndrome de Tourette, de la dystonie, de l'hémiballisme, de la chorée et des tics.

4. Composition pharmaceutique à utiliser selon l'une quelconque des revendications 1 à 3, dans laquelle la composition pharmaceutique comprend une formulation à libération prolongée de l'inhibiteur de VMAT2.

5. Composition pharmaceutique à utiliser selon l'une quelconque des revendications 1 à 4, dans laquelle l'inhibiteur de VMAT2 est l'ester (2R,3R,11bR)-3-isobutyl-9,10-diméthoxy-1,3,4,6,7,11b-hexahydro-2H-pyrido[2,1-a]isoquinolin-2-ylique d'acide (S)-2-amino-3-méthylbutyrique.

6. Composition pharmaceutique à utiliser selon l'une quelconque des revendications 1 à 5, dans laquelle le sel pharmaceutiquement acceptable est le sel de dichlorhydrate ou le sel de ditosylate.

7. Composition pharmaceutique à utiliser selon l'une quelconque des revendications 1 à 6, dans laquelle la composition se présente sous la forme d'une poudre, d'un granulé, d'une pilule, d'un comprimé, d'une capsule, d'un liquide, d'un sirop, d'une suspension ou d'une émulsion convenant à une administration orale.

8. Composition pharmaceutique à utiliser selon l'une quelconque des revendications 1 à 6, dans laquelle la composition est administrée au sujet humain qui en a besoin par la voie orale, parentérale, entérale, rectale, intranasale, buccale, sublinguale, intramusculaire ou transdermique.
